(19) Europäisches Patentamt
European Patent Office
Office européen des brevets

(11) EP 4 813 198 A1

(12) **EUROPEAN PATENT APPLICATION**
published in accordance with Art. 153(4) EPC

(43) Date of publication:
**30.09.2026 Bulletin 2026/40**

(21) Application number: **24894309.4**

(22) Date of filing: **16.08.2024**

(51) International Patent Classification (IPC):
***A01H 1/06*** $^{(2006.01)}$ ***C12N 15/82*** $^{(2006.01)}$
***A01H 5/10*** $^{(2018.01)}$

(52) Cooperative Patent Classification (CPC):
**A01H 1/06; A01H 5/10; C12N 15/82**

(86) International application number:
**PCT/KR2024/012205**

(87) International publication number:
**WO 2025/110412 (30.05.2025 Gazette 2025/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.11.2023 KR 20230161858**

(71) Applicant: **POSTECH Research and Business Development Foundation**
**Pohang-si, Gyeongsangbuk-do 37673 (KR)**

(72) Inventors:
- **CHOI, Kyuha**
  **Pohang-si Gyeongsangbuk-do 37673 (KR)**
- **KIM, Jaeil**
  **Pohang-si Gyeongsangbuk-do 37673 (KR)**
- **KIM, Heejin**
  **Pohang-si Gyeongsangbuk-do 37673 (KR)**

(74) Representative: **Wichmann, Hendrik**
**Wuesthoff & Wuesthoff**
**Patentanwälte und Rechtsanwalt PartG mbB**
**Schweigerstraße 2**
**81541 München (DE)**

Remarks:
The complete document including Reference Table(s) and the Sequence Listing(s) can be downloaded from the EPO website

# (54) J3 CHAPERONE THAT INCREASES CHROMOSOMAL CROSSOVER RECOMBINATION DURING MEIOSIS IN PLANT AND USE THEREOF

(57) The present invention relates to the J3 chaperone for increasing chromosomal crossover recombination during meiosis in plants, and uses thereof. More specifically, it concerns a method for increasing the number of crossover recombination events between homologous chromosomes during meiosis in a plant cell by inhibiting the function of the J3 chaperone protein. By using the method for increasing the number of crossover events of the present invention, crop breeding can be accelerated, and the construction of quantitative trait loci maps can be significantly expedited.

[Figure 2]

a
b
c
d
e
f
420
CTL5.14
cM
Col
hcr3
Transcript levels of J3

EP 4 813 198 A1

## Description

TECHNICAL FIELD

The present invention relates to a J3 chaperone for increasing meiotic chromosomal crossover recombination in plant and uses thereof. More specifically, the present invention relates to a method for increasing the number of crossover recombination events between homologous chromosomes during meiosis in a plant cell by inhibiting expression of an *Arabidopsis thaliana*-derived J3 protein, inserting T-DNA into a gene encoding the J3 protein to induce a loss-of-function mutant, or inducing loss of function of the endogenous J3 protein present in wild-type plants by expressing a dominant-negative mutant gene of the J3 protein.

The present invention was carried out with support from the Rural Development Administration of South Korea, the Samsung Future Technology Development Foundation, and the Suh Kyung-Bae Science Foundation (Project Nos. PJ013370012020, SSTF-BA2202-09, and SUHF-17020079).

BACKGROUND ART

Meiotic recombination is initiated by the formation of programmed DNA double-strand breaks (DSBs) and is generated during the process in which the breaks are repaired using homologous chromosomes as templates to produce crossovers or non-crossovers. Despite the formation of more than 200 DSBs, only a very small fraction proceeds to crossovers, resulting in one or two crossovers per chromosome pair.

Two pathways by which crossovers occur, namely Class I and Class II, have been known. The Class I pathway promotes the majority of crossovers and is mediated by ZMM proteins (Zip1-4, Mer3, and Msh4-5), which are crossover-promoting factors, and by the MLH1-MLH3 heterodimer endonuclease (MutL$\gamma$). The ZMM proteins stabilize recombination intermediates such as displacement loops (D-loops) and double Holliday junctions and recruit MutL$\gamma$ to generate crossovers. Crossovers formed through the Class I pathway exhibit interference, a phenomenon that suppresses the formation of additional crossovers in adjacent regions. In contrast, crossovers generated via the Cass II pathway are non-interfering and involve the endonuclease MUS81. Non-interfering crossovers are limited by several crossover suppressors, including the plant helicases FANCM and RECQ4A/4B. Among the ZMM proteins, the ZIP3/HEI10 (Human Enhancer of Invasion-10) family, which functions as a SUMO and/or ubiquitin E3 ligase, plays an important role in regulating the number and positions of interference-sensitive crossovers in a dosage-dependent manner. HEI10 begins to localize on chromosomes at early leptotene and gradually accumulates along the synaptonemal complex (SC), a proteinaceous structure formed between homologous chromosomes. In *Arabidopsis thaliana*, the HEI10 E3 ligase diffuses along the SC and localizes as hundreds of small foci together with recombination intermediates during early synapsed pachytene. Similar to biomolecular condensates exhibiting liquid-liquid phase separation, HEI10 protein becomes concentrated at designated crossover sites as one or a few large condensates during late pachytene at the expense of smaller condensates. To explain these dynamics, crossover number control, and interference, a diffusion-mediated HEI10 coarsening model has been proposed. In *Arabidopsis*, mutants lacking the function of ZYP1, a protein that forms the transverse filament structure of the SC, show loss of the interference and an increase in Class I crossovers. HORMA domain proteins, which are axial elements of the SC, are required for promoting Class I crossovers and mediating the interference. Crossover interference is limited in *Arabidopsis* by HCR1 (HIGH CROSSOVER RATE1, PROTEIN PHOSPHATASE X 1), which potentially dephosphorylates HEI10. Consistently, post-translational modifications of crossover-promoting factors, such as phosphorylation, SUMOylation, ubiquitination, and proteasome-mediated protein degradation, are involved in controlling meiotic recombination in *Saccharomyces cerevisiae*, *Schizosaccharomyces pombe*, *Caenorhabditis elegans*, and mice. However, the molecular mechanisms that mediate the protein degradation and dynamics of HEI10, a major crossover-promoting factor among plant crossover regulators, remain largely unknown.

The limited number of chromosomal recombination events occurring during meiosis acts as a major obstacle in plant breeding in terms of the time and cost required to obtain useful trait combinations. In the present invention, a genetic screening was performed to identify novel crossover suppressor factors using an *Arabidopsis thaliana* fluorescent seed crossover measurement system.

Meanwhile, Korean Patent Registration No. 2410996 discloses a "Protein phosphatase 4 complex for increasing meiotic chromosomal crossover recombination in plant cells, and uses thereof"; however, it does not describe the "J3 chaperone for increasing meiotic chromosomal crossover recombination in plant and uses thereof" of the present invention.

DETAILED DESCRIPTION

TECHNICAL PROBLEMS TO BE SOLVED

The present invention has been derived in response to the above-described needs. Inventors of the present invention screened an *Arabidopsis thaliana* fluorescent seed reporter line treated with EMS (ethyl methanesulfonate) and secured an *hcr3* (*high crossover rate 3*) mutant exhibiting an increased crossover frequency compared to the wild type. Through SHOREmap analysis, it was found that *HCR3* is a gene encoding a J3 chaperone, which belongs to the HSP40 protein family conserved from bacteria to plants and across all eukaryotes. In addition, when the *hcr3* mutant was crossed with other types of fluorescent seed or pollen reporter lines or used to generate hybrid progeny followed by GBS (genotyping by sequencing) analysis, an increased crossover frequency was shown across the entire genome. Analysis of the expression levels of crossover regulatory factors by Western blotting revealed that hcr3 and $J3^{G155R}$ (a dominant-negative mutant form of the J3 protein) limit crossover number by regulating, at the protein level, the HEI10 E3 ligase, whose expression level is proportional to crossover frequency. Based on these results, the present invention was completed by finding that the J3 of the present invention functions as a novel crossover suppressor that regulates crossover regulatory factors at the protein level and is capable of increasing crossover frequency through loss-of-function or dominant-negative mutation.

TECHNICAL MEANS FOR SOLVING PROBLEMS

To solve the above problem, the present invention provides a method for increasing the number of crossover recombination events between homologous chromosomes during meiosis in a plant cell, comprising inhibiting the function of a J3 chaperone protein in a plant cell.

The present invention further provides a method for producing a transgenic plant exhibiting an increased number of crossover recombination events between homologous chromosomes compared to the wild type, comprising inhibiting expression of a gene encoding the J3 chaperone protein, inserting T-DNA into a gene encoding the J3 chaperone protein to induce a loss-of-function mutant, or increasing expression of a gene encoding a dominant-negative J3 chaperone mutant protein in a plant cell.

The present invention further provides a transgenic plant, produced by the above method, that exhibits an increased number of crossover recombination events between homologous chromosomes compared to the wild type, and a transgenic seed thereof.

The present invention still further provides a composition for increasing the number of crossover recombination events between homologous chromosomes during meiosis in a plant cell, comprising a substance that inhibits the function of a J3 chaperone protein as an active ingredient.

ADVANTAGEOUS EFFECT OF THE INVENTION

The *Arabidopsis thaliana J3* gene according to the present invention functions as a meiotic crossover suppressor gene, so inducing a loss-of-function mutation or expressing a dominant-negative mutation increases the number of crossovers per chromosome. Even if a crop possesses two or more homologs of *J3*, expression of a dominant-negative mutation can inhibit the normal function of all *J3* homologs without inducing loss-of-function mutation in each, thereby effectively increasing the crossover number. The increase in crossover number shortens the breeding cycle and can accelerate the construction of quantitative trait loci maps, so that the method of the present invention can be advantageously applied in the field of agriculture.

BRIEF DESCRIPTION OF DRAWINGS

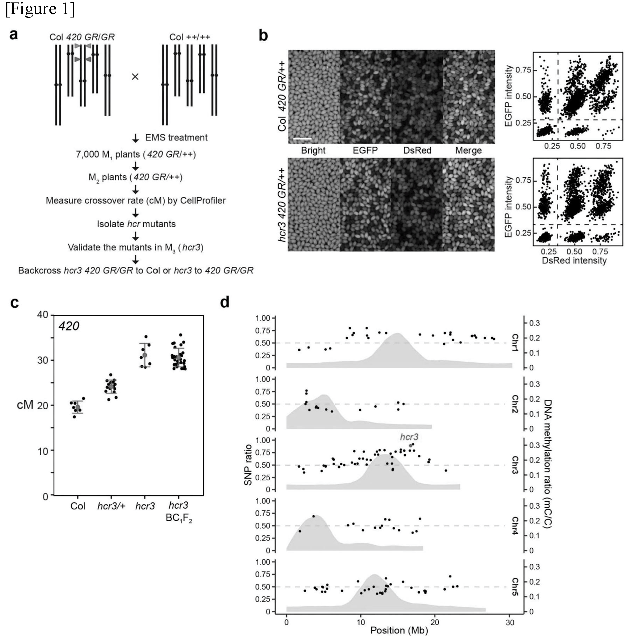

Figure 1 illustrates the process of obtaining an *Arabidopsis thaliana hcr3* (*high crossover rate* 3) mutant exhibiting increased crossovers through genetic screening using a fluorescent seed reporter line (*420 GR*/*GR*). Figure 1a shows the process of generating a screening population by treating *420 GR*/++ plants with EMS (ethyl-methanesulfonate). Figure 1b shows a representative image of the fluorescent seed line (*420 GR*/++) used for crossover-increased mutant screening, analyzed using the image analysis program CellProfiler. Figure 1c shows the measured results of crossover frequencies of *hcr3*/+, *hcr3*, and *hcr3* $BC_1F_2$. The *420 (GR*/*GR*) line is a fluorescent tagged line (FTL) in which green (eGFP) and red (dsRed) markers are located at the sub-telomeric region of chromosome 3 at an interval of 5.1 Mbp, and the genetic background is Columbia (Col). Figure 1d shows the sequencing of the genomes of 50 $BC_1F_2$ individuals to identify the mutation location of *hcr3*, indicating that it is located on chromosome 3.

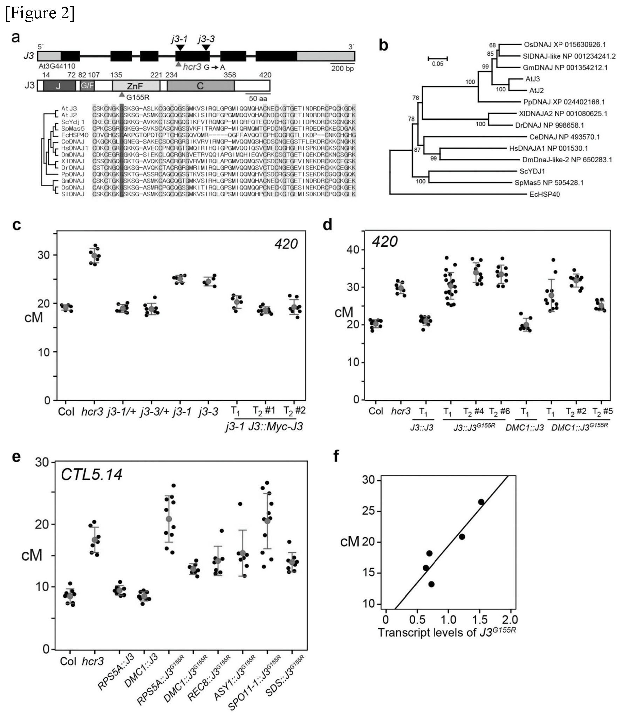

Figure 2a shows that the *hcr3* mutation occurred in the fourth exon of the *At3g44110* gene encoding the J3

chaperone (G→A). Figure 2b shows a phylogenetic tree of J3, which is evolutionarily well conserved. Figure 2c shows that introducing *J3* genomic DNA into the *j3-1* mutant restored the previously increased crossover frequency to the wild-type level. Figure 2d shows that introduction of *hcr3* into the wild type resulted in an increased crossover frequency, indicating that the *hcr3* mutation exhibits a dominant-negative property.

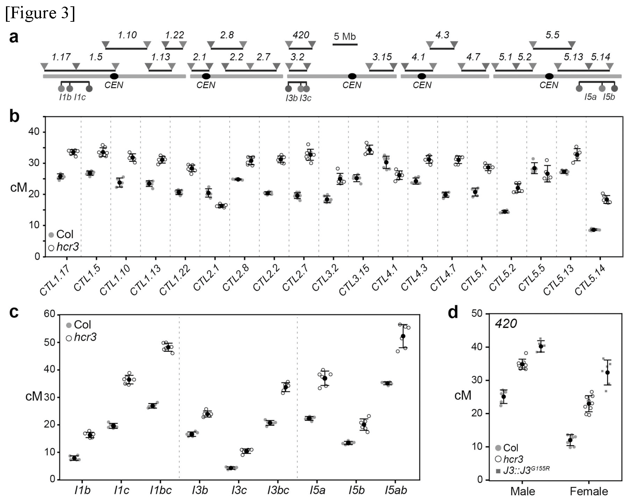

Figure 3a shows the positions of the fluorescent seed and pollen reporter lines used for crossing. Figure 3b shows that the number of crossovers increased in crosses between *hcr3* and various fluorescent seed reporter lines compared to crosses with the wild type. Figure 3c shows that the number of crossovers increased in crosses between *hcr3* and various fluorescent pollen crossover reporter lines compared to crosses with the wild type. Figure 3d shows the increase in sex-specific crossover rates in the *420* fluorescent seed reporter line.

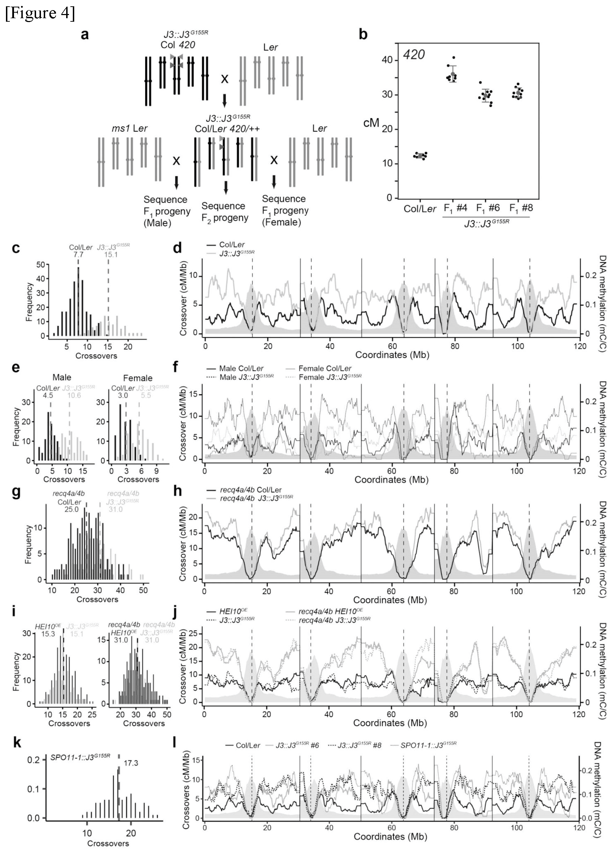

Figure 4 shows genome-wide increases in crossovers when the *hcr3* dominant-negative mutation is ectopically expressed in hybrid generations. Figure 4a shows the preparation of $F_2$ plants for GBS (genotyping-by-sequencing) analysis to construct crossover maps. Figure 4b shows that crossovers increased during meiosis in J3:J3$^{G155R}$ Col/Ler hybrid $F_1$ plants. Figure 4c shows that the average number of crossovers per J3:J3$^{G155R}$ Col/Ler $F_2$ individual increased to 15.1 compared to 7.7 in the wild type. Figure 4d shows that the increase in crossovers in J3:J3$^{G155R}$ Col/Ler was greater from the centromere toward the telomere regions. Figures 4e and 4f show that crossover rates approximately doubled in both male and female gametes. Figures 4g and 4h show that additional increases in crossover number occur when the J3 dominant-negative mutation is expressed in *recq4a/b* loss-of-function mutants, compared to the *recq4a/b* loss-of-function mutants alone. Figures 4i and 4j show that the pattern of increased crossover frequency in *hcr3* is similar to that observed when *HEI10* is overexpressed.

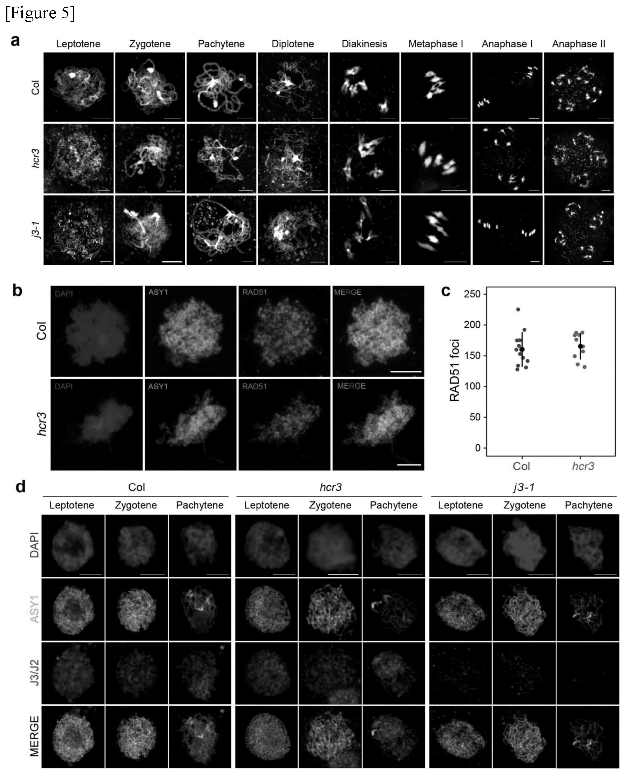

Figure 5a shows that there are no major changes in chromosome behavior during meiosis in *hcr3*. Figures 5b and 5c show that the number of RAD51 foci, which mark DSB sites, is unchanged. Figure 5d shows that the axis element ASY1 is unchanged in both *hcr3* and *J3* loss-of-function mutants.

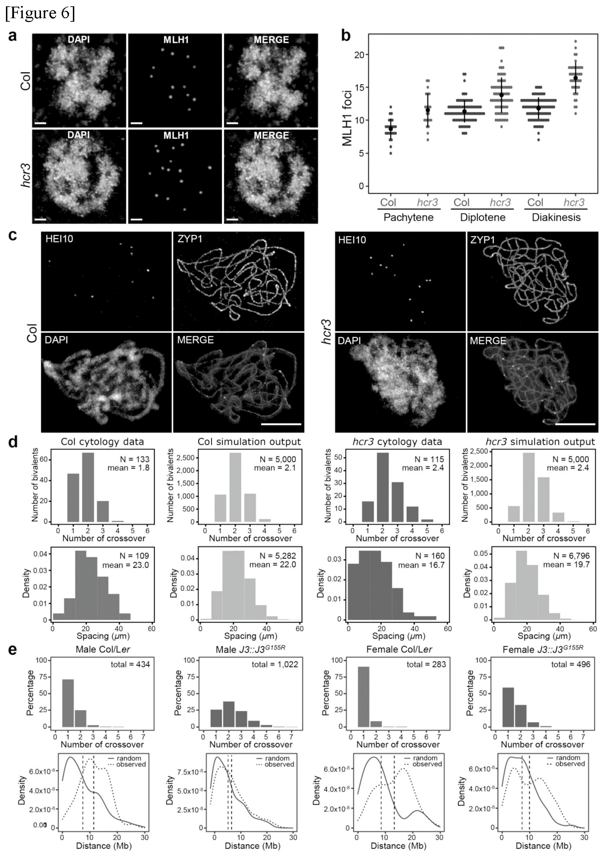

Figures 6a and 6b show that the number of MLH1 foci overlapping crossover sites is increased in *hcr3*. Figures 6c and 6d show that the number of HEI10 foci during late pachytene is increased by approximately 30% in *hcr3* compared to the wild type. In addition, the distance between two HEI10 foci on a single chromosome is closer in *hcr3* than in the wild type. Figure 6e shows that, as a result of the GBS analysis, the distances between two crossover sites on a single chromosome in *hcr3* approach a random distribution, indicating a weakening of crossover interference.

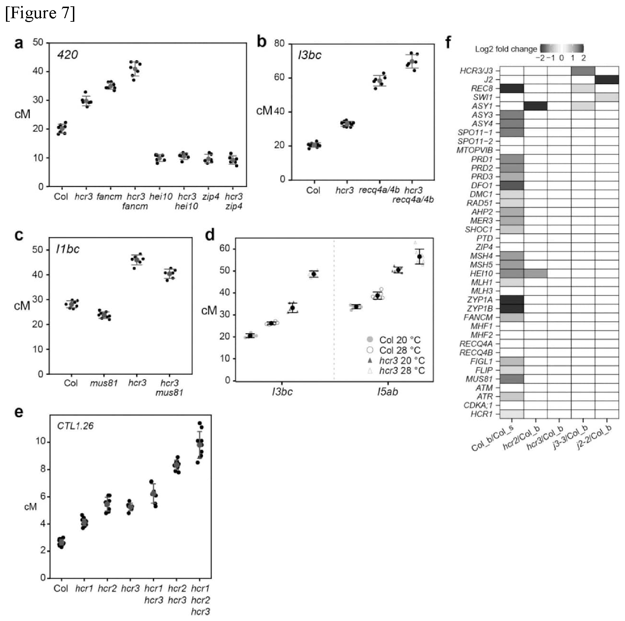

Figure 7a shows that when *hcr3* is combined with *fancm* in a double mutant, crossovers increase further compared to single mutants. It also shows that when *hcr3* is combined with *zip4* or *hei10* in double mutants, the crossover frequency is similar to that of *zip4* or *hei10* single mutants. Figure 7b shows that crossovers are further increased in *hcr3* and *recq4a/b* double mutants compared to the single mutants. Figure 7c shows that when *hcr3* is combined with *mus81* in a double mutant, the reduction in crossover frequency relative to the *hcr3* single mutant is similar to the difference between the wild type and *mus81* single mutant. Figure 7d shows that under hightemperature conditions (28°C), *hcr3* exhibits a greater increase in crossover frequency compared to the wild type. Figure 7e shows additional increases in crossover frequency in *hcr3* double and triple mutants with the previously identified Class I crossover suppressors *hcr1* and *hcr2*. Figure 7f shows the results of RNA sequencing visualized as a heatmap.

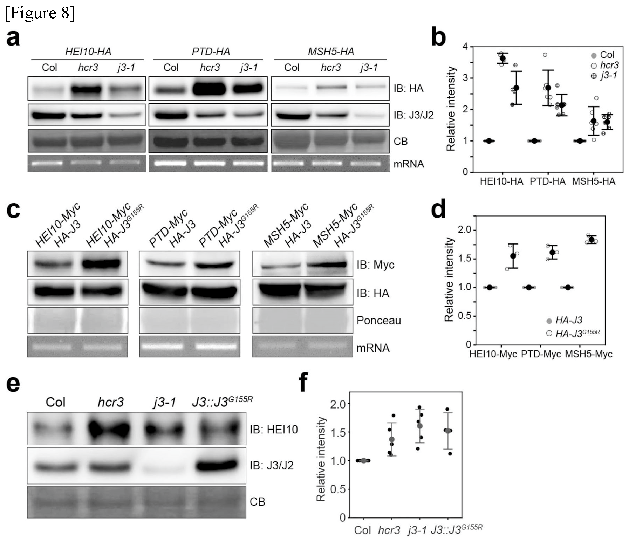

Figures 8a and 8b show Western blot results indicating that expression of HEI10-HA, PTD-HA, and MSH5-HA proteins in *Arabidopsis thaliana* protoplasts results in higher protein levels in *hcr3* and *j3-1* genotypes. Figures 8c and 8d show that in flower buds containing meiocytes, HEI10-Myc, PTD-Myc, and MSH5-Myc proteins are detected at higher level when the J3 dominant-negative mutation is coexpressed, compared to J3 wild type, as determined by Western blot. Figures 8e and 8f show that in flower buds containing meiocytes, HEI10 protein levels are higher in *hcr3*, *j3-1*, and J3:J3$^{G155R}$ genotypes compared to the wild type, as determined by Western blot.

## BEST MODE(S) FOR CARRYING OUT THE INVENTION

To achieve the object of the present invention, the present invention provides a method for increasing the number of crossover recombination events between homologous chromosomes during meiosis in a plant cell, comprising inhibiting the function of a J3 chaperone protein in a plant cell.

Homologous chromosomes refer to chromosomes that pair with each other within the cell during meiosis, each inherited from one parent, and having almost the same size and shape. During prophase I of meiosis, crossover occurs in which portions of paired homologous chromatids are exchanged (recombined), and such crossover between homologous chromosomes contributes to the generation of genetic diversity.

In the method for increasing the number of crossover recombination events according to the present invention, the inhibition of the function of a J3 chaperone protein may be achieved by inhibiting expression of a gene encoding the J3 chaperone protein, inserting T-DNA into a gene encoding the J3 chaperone protein to induce a loss-

of-function mutant, or increasing expression of a gene encoding a dominant-negative J3 chaperone mutant protein, but it is not limited thereto.

Inhibition of expression of a gene encoding the J3 protein according to the present invention can be performed by inserting the J3 protein-coding gene into an expression-inhibiting vector, such as a VIGS (virus-induced gene silencing) vector or an RNAi vector, and more preferably, may be achieved by transforming into a plant cell a recombinant vector that mediates silencing of the gene encoding the J3 protein, specifically during meiosis, but it is not limited thereto.

In the method for increasing the number of crossover recombination events according to the present invention, the J3 chaperone protein may consist of the amino acid sequence of SEQ ID NO: 3, and the dominant-negative J3 chaperone mutant protein may have a substitution of the 155th amino acid from glycine to arginine in the amino acid sequence of SEQ ID NO: 3, but it is not limited thereto.

As used herein, the term "dominant-negative mutation" refers to a mutation that exhibits a mutant phenotype by inhibiting the normal function of the wild-type allele product through antagonism. Such mutations generally produce gene products with altered molecular function and exhibit a phenotype that is relatively dominant or semidominant over the wild-type allele. Despite the presence of the wild-type allele, the mutation drives expression of the mutant phenotype, showing a dominant effect, while exhibiting a negative characteristic in terms of loss-of-function phenotype.

In the method for increasing the number of crossover recombination events according to the present invention, the expression of the dominant-negative J3 chaperone mutant protein-coding gene may be regulated by a constitutive promoter or a meiosis-specific promoter, but it is not limited thereto. The meiosis-specific promoter is not limited, and may be a SPO11-1 (SPORULATION 11-1) promoter or a DMC1 (DNA meiotic recombinase 1) promoter.

As used herein, the term "J3 chaperone protein" includes a protein including the amino acid sequence shown in SEQ ID NO: 3 and functional equivalents thereof. The term "functional equivalent" refers to a protein that, as a result of addition, substitution, or deletion of amino acids, has at least 70%, preferably at least 80%, more preferably at least 90%, and most preferably at least 95% sequence identity with the amino acid sequence shown in SEQ ID NO: 3, and exhibits substantially the same physiological activity as the protein shown in SEQ ID NO: 3. The term "substantially the same physiological activity" refers to the ability to increase the number of crossover recombination events between homologous chromosomes during meiosis in a plant cell relative to the wild type, i.e., non-transgenic plants, by inhibiting the function of the J3 protein.

The present invention further provides a gene encoding the J3 protein. The gene encoding the J3 protein of the present invention may include the nucleotide sequence shown in SEQ ID NO: 1 or SEQ ID NO: 2. Homologs of the nucleotide sequence are also encompassed within the scope of the present invention. Specifically, the gene may include a nucleotide sequence having at least 70%, more preferably at least 80%, more preferably at least 90%, and most preferably at least 95% sequence identity to the nucleotide sequence of SEQ ID NO: 1 or SEQ ID NO: 2. The "% sequence identity" of a polynucleotide is determined by comparing two optimally aligned sequences over a comparison region, and a portion of the polynucleotide sequence in the comparison region may include insertions or deletions (i.e., gaps) relative to the reference sequence for optimal alignment (excluding additions or deletions in the reference).

In the method for increasing the number of crossover recombination events according to the present invention, in addition to inhibiting the function of a J3 chaperone protein, the expression or function of one or more crossover suppressor proteins may also be inhibited, but it is not limited thereto. The crossover suppressor proteins may include FANCM (Fanconi anemia group M protein), RECQ4A (ATP-dependent DNA helicase Q-like 4A), RECQ4B, FIGL1 (AAA-ATPase FIDGETIN-LIKE 1), HCR1 (HIGH CROSSOVER RATE 1/PROTEIN PHOSPHATASE X1), or HCR2 (HIGH CROSSOVER RATE 2/HEAT SHOCK FACTOR BINDING PROTEIN), but are not limited thereto.

When the expression or function of the above-mentioned crossover suppressor proteins is additionally inhibited, the number of crossover recombination events between homologous chromosomes during meiosis in a plant cell can be further increased.

The present invention further provides a method for producing a transgenic plant exhibiting an increased number of crossover recombination events between homologous chromosomes compared to the wild type, comprising inhibiting expression of a gene encoding the J3 chaperone protein, inserting T-DNA into a gene encoding the J3 chaperone protein to induce a loss-of-function mutant, or increasing expression of a gene encoding a dominant-negative J3 chaperone mutant protein in a plant cell.

In the production method of the present invention, the J3 chaperone protein consists of the amino acid sequence of SEQ ID NO: 3, and the dominant-negative J3 chaperone mutant protein may have a substitution of the 155th amino acid from glycine to arginine in the amino acid sequence of SEQ ID NO: 3.

In the production method of the present invention, the inhibition of expression of the J3 chaperone protein-coding gene or the increase in expression of the dominant-negative J3 chaperone mutant protein-coding gene may, without being limited thereto, preferably be regulated specifically during the meiotic stage, but it is not limited thereto.

As used herein, the term "recombinant" refers to a cell that replicates heterologous nucleic acids, expresses

the nucleic acids, or expresses a peptide, heterologous peptide, or a protein encoded by heterologous nucleic acids. A recombinant cell can express a gene or a gene fragment not found in the natural form of the cell, in either sense or antisense orientation. A recombinant cell may also express a gene found in a naturally occurring cell; however, the gene has been modified and reintroduced into the cell by artificial means.

As used herein, the term "recombinant expression vector" refers to a bacterial plasmid, phage, yeast plasmid, plant cell virus, mammalian cell virus, or other vector. Generally, any plasmid or vector may be used as long as it can replicate and be stabilized within a host. Important features of the expression vector include an origin of replication, a promoter, a marker gene, and a translation control element.

An expression vector comprising suitable transcriptional and translational regulatory signals can be constructed by methods known to those skilled in the art. Such methods include in vitro recombinant DNA techniques, DNA synthesis techniques, and in vivo recombination techniques. The DNA sequence can be operably linked to an appropriate promoter in the expression vector to direct mRNA synthesis. In addition, the expression vector may include a ribosome binding site as a translation initiation site and a transcription terminator.

A preferred example of the recombinant vector of the present invention is a VIGS vector or an RNAi vector. VIGS (Virus-induced gene silencing) refers to a phenomenon in which, after introducing a plant gene into a viral vector and infecting a plant, the expression of the corresponding endogenous plant gene is suppressed. This is a type of PTGS (Post-transcriptional gene silencing) and is characterized as post-transcriptional, involving RNA turnover, and nucleotide sequence-specific. The VIGS vector can be used as a transient expression vector that expresses the foreign gene temporarily in the plant, or as a plant expression vector that expresses the introduced foreign gene permanently in the plant. A preferred example of a plant expression vector is a Ti-plasmid vector capable of transferring its T-DNA region, or a portion thereof, into plant cells when present in a suitable host such as *Agrobacterium* tumefaciens. Other types of Ti-plasmid vectors (see EP 0 116 718 B1) are currently used to transfer hybrid DNA sequences into plant cells or to create new plants by appropriately inserting hybrid DNA into the plant genome. A particularly preferred form of the Ti-plasmid vector is the so-called binary vector as claimed in EP 0 120 516 B1 and U.S. Patent No. 4,940,838.

The expression vector preferably comprises one or more selectable markers. The marker generally refers to a nucleic acid sequence that confers a selectable trait by chemical or other means and allows transformed cells to be distinguished from nontransformed cells. Examples include herbicide resistance genes such as glyphosate or phosphinothricin resistance genes, and antibiotic resistance genes such as kanamycin, G418, bleomycin, hygromycin, or chloramphenicol, but are not limited thereto.

In the recombinant vector of the present invention, the promoter may be optionally selected from plant promoters known in the art, such as a T7 promoter, SP6 promoter, CaMV 35S promoter, actin promoter, ubiquitin promoter, pEMU promoter, MAS promoter, or histone promoter. A meiosis-specific promoter, which can specifically suppress expression of the target gene during the meiotic stage of the cell, can also be used, such as the SPO11-1 (SPORULATION 11-1) promoter or the DMC1 (DNA meiotic recombinase 1) promoter, but it is not limited thereto. The term "promoter" refers to an upstream region of a structural gene that binds RNA polymerase to initiate transcription. A "plant promoter" is a promoter capable of initiating transcription in plant cells.

In the recombinant vector of the present invention, a conventional terminator may be used. Examples thereof include the nopaline synthase (NOS) terminator, rice $\alpha$-amylase RAmy1 A terminator, and phaseolin terminator, but are not limited thereto. Regarding the necessity of a terminator, such regions are generally known to increase the reliability and efficiency of transcription in plant cells. Therefore, the use of a terminator is highly preferred in the context of the present invention.

Plant transformation refers to any method for transferring DNA into a plant. Such transformation methods do not necessarily require a regeneration and/or tissue culture period. Transformation of plant species is now common for not only dicotyledonous plants but also monocotyledonous plants. In principle, any transformation method can be used to introduce the recombinant DNA according to the present invention into suitable progenitor cells. Methods may be appropriately selected from calcium/polyethylene glycol-mediated transformation of protoplasts (Krens, FA et al., 1982, Nature 296, 72-74; Negrutiu I et al., 1987, Plant Mol Biol 8, 363-373), electroporation of protoplasts (Shillito RD et al., 1985, Bio/Technol 3, 1099-1102), microinjection into plant organs (Crossway A et al., 1986, Mol Gen Genet 202, 179-185), particle bombardment of various plant tissues (DNA- or RNA-coated) (Klein TM et al., 1987, Nature 327, 70), *Agrobacterium* tumefaciens-mediated gene transfer via infiltration or mature pollen or microspores, and (incomplete) virus-mediated infection (EP 0 301 316), among others. A preferred method according to the present invention includes *Agrobacterium*-mediated DNA delivery.

The production method of the present invention further includes a step of regenerating a transgenic plant from the transgenic plant cells. Any method known in the art can be used to regenerate a transgenic plant from transgenic plant cells.

The present invention further provides a transgenic plant, produced by the above method, that exhibits an increased number of crossover recombination events between homologous chromosomes compared to the wild type,

and a transgenic seed thereof.

In one embodiment of the present invention, the plant may be a dicotyledonous plant such as Arabidopsis, potato, eggplant, tobacco, pepper, tomato, burdock, crown daisy, lettuce, balloon flower, spinach, Swiss chard, sweet potato, carrot, water dropwort, cabbage, Chinese cabbage, mustard greens, watermelon, oriental melon, cucumber, pumpkin, gourd, strawberry, soybean, mung bean, kidney bean, pea, or the like, or a monocotyledonous plant such as rice, barley, wheat, rye, maize, sugarcane, oat, onion, or the like. Preferably, the plant is a dicotyledonous plant, more preferably Arabidopsis, but it is not limited thereto.

The present invention still further provides a composition for increasing the number of crossover recombination events between homologous chromosomes during meiosis in a plant cell, comprising a substance that inhibits the function of a J3 chaperone protein as an active ingredient.

In the composition according to the present invention, the substance that inhibits the function of a J3 chaperone protein may be one or more selected from the group consisting of a compound, a peptide, a peptide mimic, a substrate analog, an aptamer, and an antibody that specifically binds to the J3 chaperone protein; or one or more selected from the group consisting of an antisense oligonucleotide, a siRNA (small interfering RNA), a shRNA (small hairpin RNA), a miRNA (microRNA), and a ribozyme that inhibits the expression of a gene encoding a J3 chaperone protein, but it is not limited thereto.

In addition, the composition of the present invention may further comprise a substance that inhibits the function of one or more crossover suppressor proteins selected from the group consisting of FANCM (Fanconi anemia group M protein), RECQ4A (ATP-dependent DNA helicase Q-like 4A), RECQ4B, FIGL1 (AAA-ATPase FIDGETIN-LIKE 1), HCR1 (HIGH CROSSOVER RATE 1/PROTEIN PHOSPHATASE X1), and HCR2 (HIGH CROSSOVER RATE 2/HEAT SHOCK FACTOR BINDING PROTEIN), but it is not limited thereto. The substance that inhibits the function of FANCM, RECQ4A, RECQ4B, FIGL1, HCR1, and HCR2 may be an antibody or aptamer specific to each protein; or a miRNA, a siRNA, or an antisense RNA capable of inhibiting the expression of the gene encoding each protein.

Hereinbelow, the present invention is explained in greater detail in view of the Examples. However, it is evident that the following Examples are given only for exemplification of the present invention and by no means the present invention is limited to the following Examples.

EXAMPLES

## Materials and Methods

### 1. Plant materials

Arabidopsis plants were grown at 22°C, 50-60% humidity, and a 16/8-hour light/dark photoperiod. Seeds were incubated at 4°C in the dark for 3-4 days for germination. FTL (Fluorescent Tagged Line) lines expressed in seeds and pollen were used in the present invention (Wu, G., et al., (2015) Genetics 200:35-45; Melamed-Bessudo, C., et al., (2005) Plant J. 43:458-66). T-DNA insertion lines *j3-1* (SALK_132923), *zip4-2* (SALK_068052), *mus81-2* (SALK_107515), *hei10-2* (SALK_014624), and the EMS mutant *fancm*-1 were obtained from the NASC (Nottingham Arabidopsis Stock Centre). Wild-type alleles were genotyped using the primer set j3-1_geno_F and j3-1_geno_R, and T-DNA alleles were genotyped using j3-1_geno_F and LBb1.3 primers. Genotyping of *hcr3* was performed by PCR amplification using *hcr3*_dCAPS_BamHI_F and *hcr3*_dCAPS_R followed by *Bam*HI restriction enzyme digestion. Genotyping of *zip4-2, mus81-2*, and *fancm*-1 was performed as previously described (Yelina, N. E. et al., (2015) Genes Dev. 9:2183-202; F. Hartung et al., (2006), Nucleic Acids Research, 34(16):4438-4448).

[Table 1]

| Information of primers used in the present invention | |
|---|---|
| Primer | Sequence (5'-3') (SEQ ID NO:) |
| *hcr3* dCAPS BamHI F | CACATTTCAAGGAGGCTCCAGATTTGGATC (4) |
| *hcr3* dCAPS R | GCTATATGTGTCCTAGGTTCAT (5) |
| LBb1.3 | ATTTTGCCGATTTCGGAAC (6) |
| *j3-1*_geno F | TGTTGAATTTTGATCCGATCTG (7) |
| *j3-1*_geno_R | CCCTTTCTCTTGAACTTTGGG (8) |
| pPZP211-gJ3_GA_F | GCCTGCAGGTCGACTCTAGAGAAATCAGTGAT GATCAAATTACTTAC (9) |

(continued)

| Information of primers used in the present invention | |
|---|---|
| Primer | Sequence (5'-3') (SEQ ID NO:) |
| pPZP211-gJ3_GA_R | AGGCGCGCCCTCGAGTTGAACTCTTTTTAAAATACAACTGGTATG (10) |
| pJ3(2.2 kb)_Lv0_F(GGAG) | CCGAAGACGGCTCAGGAGCCAACCTACTTGCATGTTAACAAGAAACTATTATTC (11) |
| pJ3_Lv0_R(CCAT) | CCGAAGACGGCTCGATGGCTTTTCGCCGTTGTAACGAAAAC (12) |
| J3_Lv0_F(AATG) | AAAGAAGACAACTCAAATGATGTTCGGTAGAGGACCCTC (13) |
| J3 Lv0_R(TTCG) | AAAGAAGACAAACCTTTACTGCTGGGCACATTGC (14) |
| J3(G155R) SDM F | ATTGCAGAAAGAGATCAAAATCTGGAGCC (15) |
| J3(G155R) SDM R | GGCTCCAGATTTTGATCTCTTTCTGCAAT (16) |
| J3-Ter_Lv0_F(GCTT) | CCGAAGACGGCTCAGCTTGTAACTCCTTAGAGAGACTTTGAC (17) |
| J3-Ter_Lv0_R(CGCT) | CCGAAGACGGCTCGAGCGAACTCTTTTTAAAATACAACTGGTATG (18) |
| pDMC1(1.5kb)_Lv0_F(G GAG) | CCGAAGACGGCTCAGGAGAAAGAAACCAAAGTTCCATGTCCAT (19) |
| pDMC1_Lv0_R(AATG) | CCGAAGACGGCTCGCATTCCGATCACTGACACAAGCAAAAATAAA (20) |
| HEI10_Lv0_GA_F(AATG ) | CCCGAAGACGGCTCAAATGATGAGATGCAACGCGTGTTGGA (21) |
| HEI10_Lv0_GA_R(ggTT CG) | CCGAAGACGGCTCGCGAACCTAGCGTGAACAGCTGAGGGCGGGAA (22) |
| MSH5_Lv0_GA_F(AATG ) | CCGAAGACGGCTCAAATGATGGAGGAAATGGAGGACACTG (23) |
| MSH5_Lv0_GA_R(ggTT CG) | CCGAAGACGGCTCGCGAACCGGAAGTGAAGATATCTTGAAAG (24) |
| PTD_Lv0_GA_F(AATG) | CCGAAGACGGCTCAAATGATGGCGACGGCGGGATCA (25) |
| PTD_Lv0_GA_R(ggTTC G) | CCGAAGACGGCTCGCGAACCTCAGTTGAATTTGGGACTGAG (26) |
| pRPS5A_Lv0_F(GGAG) | CCGAAGACGGCTCAGGAGCCATAATCGTGAGTAGATATATTACTCAAC (27) |
| pRPSSA Lv0 R(AATG) | CCGAAGACGGCTCGCATTGGCTGTGGTGAGAGAAACAGAGCGTGAGCTC (28) |

(continued)

| Information of primers used in the present invention | |
|---|---|
| Primer | Sequence (5'-3') (SEQ ID NO:) |
| pREC8_Lv0_F(GGAG) | CCCGAAGACGGCTCAGGAGATGGAGGTAGCG GGATAATTGA (29) |
| pREC8_Lv0_R(AATG) | CCCGAAGACGGCTCGCATTCCCGCGTGCAATG TAGCGGCCATCCTTAAGAAGAAGAAA (30) |
| pASY1(1.3kb)_Lv0_F(GG AG) | CCCGAAGACGGCTCAGGAGTGGCAGGATATAT TGTGGTG (31) |
| pASY1_Lv0_R(AATG) | CCCGAAGACGGCTCGCATTCCTTTTGCAGAAG TGTGAAACGA (32) |
| pSPO11-1_Lv0_F(GGAG) | CCCGAAGACGGCTCAGGAGGACCTCTCTGTTC TTAATTCC (33) |
| pSPO11-1_Lv0_R(AATG) | CCCGAAGACGGCTCGCATTCCCTCTTTCGAGTT TCAAAACTGAAA (34) |
| pSDS_Lv0_F(GGAG) | CCCGAAGACGGCTCAGGAGTGGCAGGATATAT TGTGGTG (35) |
| pSDS_Lv0_R(AATG) | CCCGAAGACGGCTCGCATTCCTTTTTCTCCGTA CGAAAGCTTGAAA (36) |

### 2. Induction of mutation by EMS (ethyl methanesulfonate) treatment

Approximately 10,000 seeds of *420* GR/++ hemizygote plants obtained from crossing *420* (GR/GR) homozygotes with wild-type Arabidopsis (Col-0) were soaked in 40 mL of 100 mM phosphate buffer (pH 7.5) for 1 hour, rinsed with fresh phosphate buffer, treated with 0.3% (v/v) EMS, and incubated at room temperature for 12 hours (Figure 1a). EMS-treated seeds were washed 10 times with distilled water and immediately sown in soil. Approximately 7,000 $M_1$ plants germinated and grew from those seeds. Seeds from 12 independent $M_1$ plants were pooled to generate approximately 600 $M_2$ populations. From each $M_2$ population, approximately 150 seeds were selected based on red and green fluorescence, designated as *420* GR/++ hemizygotes, grown, and self-fertilized. Seeds derived therefrom were used for *420* crossover frequency analysis.

### 3. Measurement of crossover frequency and interference using fluorescent seeds and fluorescent pollen

Crossover frequency was analyzed by counting fluorescent and non-fluorescent seeds of FTL/++ hemizygous plants using the CellProfiler image analysis program. CellProfiler quantified the number of green-only fluorescent seeds ($N_{Green}$), red-only fluorescent seeds ($N_{Red}$), and the total number of seeds ($N_{Total}$), and crossover frequency (cM) was calculated using the following formula. Significance of crossover frequency between genotypes was assessed using Welch's t-test.

$$cM = 100 \times (1-[1-2 \times (N_{Green}+N_{Red})/N_{Total}]^{1/2})$$

Pollen FTLs were generated in a *qrt-1* mutant background, in which the four pollen products of male meiosis remain attached. FTLs expressed eYFP (Y), dsRed (R), or eCFP (C) under the control of the *LAT52* promoter. Measurement of crossover frequency and interference based on pollen tetrads of FTLs was performed using DeepTetrad (Berchowitz, L. E. & Copenhaver, G. P., 2008, Nat. Protoc. 3:41-50; Lim, E. C. et al., 2020, Plant J. 101:473-483). DeepTetrad is a deep-learning-based image analysis system capable of recognizing the types of pollen tetrads for FTL intervals.

FTL intervals *(I1bc, I1fg, I2fg, I3bc,* and *I5ab*) produce twelve tetrad types: no recombination (A), single

crossover interval 1 (B; SCO-i1), single crossover interval 2 (C; SCO-i2), two-strand double crossover (D; 2st DCO), three-strand double crossover a (E; 3st DCOa), three-strand double crossover b (F; 3st DCOb), four-strand double crossover (G; 4st DCO), non-parental ditype interval 1, non-crossover interval 2 (H; NPD-i1 NCO-i2), non-crossover interval 1, non-parental ditype interval 2 (I; NCO-i1 NPD-i2), non-parental ditype interval 1, single crossover interval 2 (J; NPD-i1 SCO-i2), single crossover interval 1, non-parental ditype interval 2 (K; SCO-i1 NPD-i2), and non-parental ditype interval 1, non-parental ditype interval 2 (L; NPD-i1 NPD-i2). Fluorescent seed status was determined using DeepTetrad, and crossover frequency and interference were calculated using Perkins' equation.

The crossover interference rate (IFR=$\sigma$) between two linked intervals was calculated, based on the following formula, by using DeepTetrad as the ratio of the genetic map distance including an adjacent crossover ($X_\gamma$) to the genetic map distance excluding an adjacent crossover ($X_\delta$).

$$X_\gamma = \frac{0.5T_\gamma + 3NPD_\gamma}{PD_\gamma + T_\gamma + NPD_\gamma} = \frac{0.5(D+E+F+G+K)+3(J+L)}{(C+I)+(D+E+F+G+K)+(J+L)}$$

$$X_\delta = \frac{0.5T_\delta + 3NPD_\delta}{PD_\delta + T_\delta + NPD_\delta} = \frac{0.5(B)+3(H)}{(A)+(B)+(H)}$$

$$\sigma = \frac{X_\gamma}{X_\delta}$$

**4. Determination of *hcr3* mutation using DNA sequencing and SHOREmap**

Fifty *hcr3* $BC_1F_2$ individuals exhibiting higher *420* crossover frequencies than the wild-type were identified, and 5 mg of seeds from each $BC_1F_2$ individual were collected. The sterilized seeds were germinated on 1/2 MS solid medium, and 7-day-old seedlings were harvested. Approximately 3 g of the collected seedlings were ground into powder using liquid nitrogen. The powder was homogenized in 40 mL of nuclear isolation buffer (25 mM Tris-HCl, pH 7.5; 0.44 M sucrose; 10 mM $MgCl_2$; 0.5% Triton X-100; 10 mM β-mercaptoethanol; 2 mM spermine; EDTA-free Protease Inhibitor Cocktail). The homogenate was stirred and reacted on ice for 30 min and then centrifuged at 3,000 g for 25 min at 4°C, and the pellet was used for DNA extraction using CTAB (cetyl trimethylammonium bromide). The extracted and purified DNA was sheared to 200-500 bp fragments using a Bioruptor sonicator. The purified DNA was used to construct libraries with the Illumina TruSeq Nano DNA LT Library Prep Kit, and the *hcr3* $BC_1F_2$ libraries were sequenced by using Illumina Genome Analyzer HiSeq 2000 system (100 bp paired-end).

SHOREmap (v.3.0) was employed to align the paired-end reads to the TAIR10 reference genome using GenomeMapper tool. Raw reads were trimmed based on quality values with Phred score cutoffs of +33 or +64 using the SHORE import function. Sequence variants between *hcr3* $BC_1F_2$ and the TAIR10 reference assembly were identified using the SHORE consensus function. SNPs (single nucleotide polymorphisms) with high marker scores (>40) were used for allele frequency analysis via the SHOREmap backcross function. Mutations were screened based on (i) allele frequencies of 80% or higher and (ii) predicted non-synonymous changes, splice site alterations, or premature stop codons within genes. Additionally, candidate mutations were further evaluated based on their predicted or known functions related to meiosis, nuclear protein localization, or molecular functions annotated in the TAIR database.

**5. Determination of dominant-negative allele *HCR3* of *J3***

A 4.1 kb genomic DNA fragment, including the 2.2 kb promoter and the *HCR3*/*J3* gene, was amplified using the pPZP211-gJ3_GA_F and pPZP211-gJ3_GA_R primer sets. The PCR product was cloned into the pPZP211 vector using Gibson assembly. The resulting construct was introduced into *Agrobacterium tumefaciens* strain GV3101-pSoup by electroporation, and the *Agrobacterium* culture was used to transform plants via the floral dipping method. $T_1$ plants were screened by genotyping with specific primer sets for kanamycin resistance and the *HCR3*/*J3* transgene.

**6. J3 phylogenetic tree**

The neighbor-joining method was used to construct the J3 phylogenetic tree. The amino acid information

used for multiple sequence alignment is as follows: AtJ3, AtJ2, OsDNAJ (XP_015630926.1), SlDNAJ-like (NP_001234241.2), GmDNAJ (NP_0001354212.1), PpDNAJ (XP_024402168.1), XlDNAJA2 (NP_001080625.1), DrDNAJ (NP_998658.1), CeDNAJ (NP_493570.1), HsDNAJA1 (NP_001530.1), DmDnaJ-like-2 (NP_650283.1), ScYDJ1, SpMas5 (NP_595428.1), EcHSP40.

### 7. Preparation of *HCR3*/*J3* dominant-negative mutant plant

To generate plants expressing the *HCR3* dominant-negative allele mutant, the wild-type genomic DNA including the promoters and 5'-UTRs of *J3, DMC1, RPS5A, REC8, ASY1, SPO11-1,* or *SDS* genes was amplified using forward and reverse primers and cloned into the Lv0 vector (pICH41331). The *HCR3*/*J3* genomic DNA was amplified from the genomic DNA of *hcr3* mutant plants using forward and reverse primers and cloned into the Lv0 vector (pICH41331). Each promoter and the *hcr3* Lv0 vector were assembled into the Lv1 position 2 vector (pICH47742). The resulting Lv1 vector was then assembled with a linker (pICH41744) and the BAR antibiotic resistance gene (pICSL11017) into the Lv2 binary vector (pAGM4723). The Lv2 binary vector was introduced into *Agrobacterium* strain GV3101-pSoup, and Arabidopsis plants were transformed via the floral dipping method.

### 8. Determination of GBS and crossover of $F_2$ plant

Genomic DNA was extracted from 96 J3:J3$^{G155R}$ Col/Ler $F_2$ individuals using CTAB for sequencing library preparation. 150 ng of DNA was fragmented using 0.3 units of dsDNA Shearase (Zymo Research) in a final volume of 15 μL. The fragmented DNA was end-repaired in 30 μL of reaction buffer at 20°C for 30 minutes, containing 3 units of T4 DNA polymerase (New England Biolabs), 10 units of T4 polynucleotide kinase (Thermo Fisher Scientific), 1.25 units of Klenow fragment (New England Biolabs), and 0.4 mM dNTPs. DNA fragments were purified using AMPure XP magnetic SPRI beads (Beckman-Coulter, A63881). The DNA was A-tailed and ligated with barcoded Illumina adapters in a 20 μL reaction according to previously described methods (Rowan, B. A., et al., 2015, G3 (Bethesda) 5:385-398). Eight DNA libraries were pooled, washed, and eluted in 30 μL of elution buffer (10 mM Tris-HCl, pH 8.0).

The mixture was added to a tube containing 16 μL of AMPure XP magnetic SPRI beads, incubated at room temperature for 5 minutes, and placed on a magnetic rack for 2 minutes. The supernatant (42 μL) was transferred to a new tube and mixed with 0.23× SPRI beads. After incubating at room temperature for 5 minutes, the tube was placed on a magnetic rack for 2 minutes, the supernatant removed, and the beads were washed twice with 80% ethanol for 30 seconds. Beads were air-dried for 10 minutes and DNA was eluted in 20 μL of 10 mM Tris (pH 8.0). 12 μL of the eluted DNA was PCR-amplified using the KAPA HiFi Hot-Start ReadyMix PCR kit (Kapa Biosystems) with known DNA oligonucleotides (Rowan, B. A., et al., 2015). PCR products were purified using SPRI beads and quantified with a Bioanalyzer. Sequencing of the six barcoded libraries was performed on the Illumina HiSeq X platform using paired-end 150 bp sequencing.

### 9. Cytological analysis of meiocytes in wild-type and *hcr3*

Meiotic chromosome experiments in Arabidopsis were prepared from fixed flower buds and DAPI staining as previously described (Chelysheva, L. et al., 2010, Cytogenet. Genome Res. 29:143-153). Cells at the zygotene stage were immunostained for ASY1 and J3, late pachytene cells were immunostained for HEI10, and diplotene cells were immunostained for MLH1. Meiocytes at the zygotene stage were also immunostained for ASY1 and RAD51 using fresh flower buds. Antibodies used included α-ASY1 (rat, 1:200 or 1:500 dilution), α-ZYP1 (rabbit, 1:200), α-MLH1 (rabbit, 1:200), and α-RAD51 (rabbit, 1:300). Microscopy was performed using a DeltaVision personal DV microscope (Applied Precision/GE Healthcare) equipped with a CDD Coolsnap HQ2 camera. Image capture was conducted with SoftWoRx software version 5.5. For co-immunostaining of ASY1 and RAD51 in zygotene-stage nuclei, individual cell images were acquired as 10 Z-stack optical sections at 0.2 μM intervals, and maximum intensity projections for each cell were set using ImageJ. The number of MLH1 foci per meiotic cell and the number of RAD51 foci per cell were manually scored relative to the axial protein ASY1. Welch's test was used to evaluate statistically significant differences in MLH1 and RAD51 foci between wild-type and *hcr3*.

### 10. Quantitative analysis of ZMM proteins in protoplasts and transgenic plants

Protoplast transient expression vector was constructed using Golden Gate cloning. Genomic DNA of *HCR3*/J3, *HEI10, PTD,* and *MSH5* amplified by PCR was cloned into the Lv0 universal vector (pICH41331). For epitope tagging, an Lv0 vector containing a coding region lacking a stop codon was assembled into an Lv1 vector (pICH4742) together with 35S promoter vector (pICH51266), N-terminal or C-terminal tag vector (Myc tag/-pICSL50010 and HA tag/pICSL50009), and NOS terminator vector (pICH41421).

To generate transgenic plants expressing epitope-tagged proteins, the *HCR3*/*J3* Lv1 vector produced by the above method and each *HEI10, PTD,* and *MSH5 Lv1* vector were assembled into Lv2 binary vector (pAGM4723) together with a linker (pICH41744) and the *BAR* antibiotic resistance gene (pICSL11017). The completed Lv2 binary vector was introduced into *Agrobacterium* strain GV3101-pSoup and used to transform *Arabidopsis* via the floral dipping method.

Plasmid DNA and mesophyll protoplasts were prepared as previously described (Hwang, I. & Sheen, J. (2001) Nature 413:383-389). A total of $4 \times 10^4$ protoplasts were transfected with 40 μg of plasmid DNA and incubated at room temperature for 10 hours. Total proteins were extracted using extraction buffer (50 mM Tris-HCl, pH 7.5, 100 mM NaCl, 5 mM EDTA, 1 mM dithiothreitol, protease inhibitor cocktail (Roche), and 1% Triton X-100), separated by SDS-PAGE on an 8% polyacrylamide gel, and transferred to a nitrocellulose membrane. Proteins were then detected using anti-HA-HRP (1:2,000, Roche 12013819001) or anti-Myc-HRP (1:2,000, Santa Cruz sc-9E10) antibodies.

For *in vivo* protein analysis, young flower buds containing meiotic cells from 5-6-week-old plants were collected (about 0.1 g) and powdered in liquid nitrogen. Total proteins were extracted using solubilization buffer (25 mM HEPES, 5 mM EDTA, 2% SDS), and subsequent procedures were performed as described above.

**Example 1. Crossover increase through *HCR3*/*J3* mutation**

Through previous studies, it has been established that an increase in number of crossover frequency in plants can be achieved either by mutation in crossover-suppressing genes (*FANCM*, *RECQ4A*/*B, FIGL1, HCR1*/*PPX1*) or by overexpression of crossover-promoting genes (*HEI10*). In the present invention, EMS was applied to fluorescent seed crossover measurement lines to identify new crossover-suppressing genes, and mutants exhibiting increased crossover were screened (Figure 1).

As a result, the *hcr3* mutant with elevated crossover frequency was obtained, and next-generation sequencing (NGS) showed that the increased crossover in this mutant was caused by a mutation occurred in the *At3g44110* gene encoding the J3 chaperone (Figure 2). Loss-of-function mutants of *J3* generated via T-DNA insertion into *J3* gene also exhibited increased crossover (Figure 2c), and expression of the dominant-negative allele of *J3* was shown to inhibit the function of endogenous *J3* homologs in the plant, producing an additional increase in crossover beyond that observed in single-gene loss-of-function mutants (Figure 2d). Although previous studies reported examples of regulating crossover frequency through changes in transcript levels of crossover-regulating genes, the present invention is the first to demonstrate a phenotype of increased crossover by directly modulating the levels of crossover-regulating proteins during meiosis.

**Example 2. Crossover increase via meiosis-specific expression of *HCR3* gene**

The J3 chaperone contributes to protein stabilization under conditions unsuitable for plant growth, such as high temperatures. Loss-of-function mutants of *J3* exhibit reduced ability to respond to environmental changes, and double mutants with its homolog *J2* are lethal during early development. Therefore, to specifically express the dominant-negative mutant form of *J3* during meiosis, meiosis-specific promoters were used to examine patterns of the increased crossover frequency (Figure 2e). When the *SPO11-1* promoter was used, plants showed higher increases in crossover frequency than with the endogenous *J3* promoter, and this pattern indicated that the crossover frequency increased proportionally with the expression level of the dominant-negative *J3* mutant (Figure 2f). Furthermore, when the dominant-negative *J3* mutant was expressed in hybrid generations using the *SPO11-1* promoter, crossover frequency was also higher than with the endogenous *J3* promoter (Figures 4k and 4l). These results demonstrate that the magnitude of the increase in recombination frequency can be modulated by controlling the timing and level of expression of the dominant-negative *J3* mutant using different promoters.

**Example 3. Additional increase in number of crossovers through double mutants of crossover suppressor genes and *hcr3***

It was found that, when *hcr3* was combined in double or triple mutants with previously known Class II crossover suppressor genes (*FANCM, RECQ4A*/*4B*) or Class I crossover suppressor genes (*HCR1, HCR2*), the number of crossovers increased further compared to single mutants (Figure 7e). This is significant in that it allows for an additional increase in crossover number beyond that achievable by previously established methods for increasing crossover frequency.

## Claims

1. A method for increasing the number of crossover recombination events between homologous chromosomes during meiosis in a plant cell, the method comprising inhibiting the function of a J3 chaperone protein in a plant cell.

2. The method according to Claim 1, wherein the inhibiting the function of a J3 chaperone protein is achieved by inhibiting expression of a gene encoding the J3 chaperone protein, inserting T-DNA into a gene encoding the J3 chaperone protein to induce a loss-of-function mutant, or increasing expression of a gene encoding a dominant-negative J3 chaperone mutant protein in a plant cell.

3. The method according to Claim 2, wherein the J3 chaperone protein consists of the amino acid sequence of SEQ ID NO: 3, and the dominant-negative J3 chaperone mutant protein has a substitution of the 155th amino acid from glycine to arginine in the amino acid sequence of SEQ ID NO: 3.

4. The method according to Claim 2, wherein the expression of a gene encoding a dominant-negative J3 chaperone mutant protein is regulated by a constitutive promoter or a meiosis-specific promoter.

5. The method according to Claim 1, wherein, in addition to inhibiting the function of a J3 chaperone protein, the expression or function of one or more crossover suppressor proteins is also inhibited.

6. The method according to Claim 5, wherein the crossover suppressor protein is FANCM (Fanconi anemia group M protein), RECQ4A (ATP-dependent DNA helicase Q-like 4A), RECQ4B, FIGL1 (AAA-ATPase FIDGETIN-LIKE 1), HCR1 (HIGH CROSSOVER RATE 1/PROTEIN PHOSPHATASE X1), or HCR2 (HIGH CROSSOVER RATE 2/HEAT SHOCK FACTOR BINDING PROTEIN).

7. A method for producing a transgenic plant exhibiting an increased number of crossover recombination events between homologous chromosomes compared to a wild type, the method comprising inhibiting expression of a gene encoding a J3 chaperone protein, inserting T-DNA into a gene encoding a J3 chaperone protein to induce a loss-of-function mutant, or increasing expression of a gene encoding a dominant-negative J3 chaperone mutant protein in a plant cell.

8. The method according to Claim 7, wherein the J3 chaperone protein consists of the amino acid sequence of SEQ ID NO: 3, and the dominant-negative J3 chaperone mutant protein has a substitution of the 155th amino acid from glycine to arginine in the amino acid sequence of SEQ ID NO: 3.

9. A transgenic plant having an increased number of crossover recombination events between homologous chromosomes compared to a wild type, produced by the method of Claim 7.

10. A seed of the transgenic plant of Claim 9.

11. A composition for increasing the number of crossover recombination events between homologous chromosomes during meiosis in a plant cell, comprising a substance that inhibits the function of a J3 chaperone protein as an active ingredient.

12. The composition according to Claim 11, wherein the substance that inhibits the function of a J3 chaperone protein is one or more selected from the group consisting of a compound, a peptide, a peptide mimic, a substrate analog, an aptamer, and an antibody that specifically binds to the J3 chaperone protein; or one or more selected from the group consisting of an antisense oligonucleotide, a siRNA (small interfering RNA), a shRNA (small hairpin RNA), a miRNA (microRNA), and a ribozyme that inhibits expression of a gene encoding the J3 chaperone protein.

13. The composition according to Claim 11, wherein the composition further comprises a substance that inhibits the function of one or more proteins selected from the group consisting of FANCM (Fanconi anemia group M protein), RECQ4A (ATP-dependent DNA helicase Q-like 4A), RECQ4B, FIGL1 (AAA-ATPase FIDGETIN-LIKE 1), HCR1 (HIGH CROSSOVER RATE 1/PROTEIN PHOSPHATASE X1) and HCR2 (HIGH CROSSOVER RATE 2/HEAT SHOCK FACTOR BINDING PROTEIN).

[Figure 1]

[Figure 2]


a
J3
5´
3´
j3-1
j3-3
At3G44110
hcr3 G → A
200 bp
14 72 82 107 135 221 234 358 420
J3
J
G/F
ZnF
C
G155R
50 aa
AtJ3
AtJ2
ScYdj1
SpMas5
EcHSP40
CeDNAJ
HsDNAJ1
DmDNAJ
XlDNAJ
DrDNAJ
PpDNAJ
GmDNAJ
OsDNAJ
SlDNAJ
b
0.05
OsDNAJ XP 015630926.1
SlDNAJ-like NP 001234241.2
GmDNAJ NP 001354212.1
AtJ3
AtJ2
PpDNAJ XP 024402168.1
XlDNAJA2 NP 001080625.1
DrDNAJ NP 998658.1
CeDNAJ NP 493570.1
HsDNAJA1 NP 001530.1
DmDnaJ-like-2 NP 650283.1
ScYDJ1
SpMas5 NP 595428.1
EcHSP40
c
420
cM
Col hcr3 j3-1/+ j3-3/+ j3-1 j3-3 T1 T2 #1 T2 #2
j3-1 J3::Myc-J3
d
420
cM
Col hcr3 T1 T1 T2 #4 T2 #6 T1 T1 T2 #2 T2 #5
J3::J3 J3::J3G155R DMC1::J3 DMC1::J3G155R
e
CTL5.14
cM
Col hcr3 RPS5A::J3 DMC1::J3 RPS5A::J3G155R DMC1::J3G155R REC8::J3G155R ASY1::J3G155R SPO11-1::J3G155R SDS::J3G155R
f
cM
Transcript levels of J3G155R

[Figure 3]

a
1.10
1.22
2.8
420
5 Mb
4.3
5.5
1.17
1.5
1.13
2.1
2.2
2.7
3.2
3.15
4.1
4.7
5.1
5.2
5.13
5.14
CEN
I1b I1c
I3b I3c
I5a I5b
b
cM
Col
hcr3
CTL1.17
CTL1.5
CTL1.10
CTL1.13
CTL1.22
CTL2.1
CTL2.8
CTL2.2
CTL2.7
CTL3.2
CTL3.15
CTL4.1
CTL4.3
CTL4.7
CTL5.1
CTL5.2
CTL5.5
CTL5.13
CTL5.14
c
I1b
I1c
I1bc
I3b
I3c
I3bc
I5a
I5b
I5ab
d
420
J3::J3G155R
Male
Female

[Figure 4]

[Figure 5]

a
Leptotene
Zygotene
Pachytene
Diplotene
Diakinesis
Metaphase I
Anaphase I
Anaphase II
Col
hcr3
j3-1
b
DAPI
ASY1
RAD51
MERGE
Col
hcr3
c
RAD51 foci
250
200
150
100
50
0
Col
hcr3
d
Col
hcr3
j3-1
Leptotene
Zygotene
Pachytene
DAPI
ASY1
J3/J2
MERGE

[Figure 6]

a
DAPI
MLH1
MERGE
Col
hcr3
b
MLH1 foci
Col
hcr3
Pachytene
Diplotene
Diakinesis
c
HEI10
ZYP1
DAPI
MERGE
d
Col cytology data
Col simulation output
hcr3 cytology data
hcr3 simulation output
N = 133
mean = 1.8
N = 5,000
mean = 2.1
N = 115
mean = 2.4
N = 5,000
mean = 2.4
Number of bivalents
Number of crossover
N = 109
mean = 23.0
N = 5,282
mean = 22.0
N = 160
mean = 16.7
N = 6,796
mean = 19.7
Density
Spacing (μm)
e
Male Col/Ler
Male J3::J3G155R
Female Col/Ler
Female J3::J3G155R
total = 434
total = 1,022
total = 283
total = 496
Percentage
Number of crossover
random
observed
Density
Distance (Mb)

[Figure 7]

a
420
cM
Col
hcr3
fancm
hcr3 fancm
hei10
hcr3 hei10
zip4
hcr3 zip4
b
I3bc
cM
Col
hcr3
recq4a/4b
hcr3 recq4a/4b
c
I1bc
cM
Col
mus81
hcr3
hcr3 mus81
d
cM
Col 20 °C
Col 28 °C
hcr3 20 °C
hcr3 28 °C
I3bc
I5ab
e
CTL1.26
cM
Col
hcr1
hcr2
hcr3
hcr1 hcr3
hcr2 hcr3
hcr1 hcr2 hcr3
f
Log2 fold change
−2 −1 0 1 2
HCR3/J3
J2
REC8
SWI1
ASY1
ASY3
ASY4
SPO11−1
SPO11−2
MTOPVIB
PRD1
PRD2
PRD3
DFO1
DMC1
RAD51
AHP2
MER3
SHOC1
PTD
ZIP4
MSH4
MSH5
HEI10
MLH1
MLH3
ZYP1A
ZYP1B
FANCM
MHF1
MHF2
RECQ4A
RECQ4B
FIGL1
FLIP
MUS81
ATM
ATR
CDKA;1
HCR1
Col_b/Col_s
hcr2/Col_b
hcr3/Col_b
j3-3/Col_b
j2-2/Col_b

[Figure 8]

a
HEI10-HA
PTD-HA
MSH5-HA
Col
hcr3
j3-1
IB: HA
IB: J3/J2
CB
mRNA
b
Relative intensity
Col
hcr3
j3-1
HEI10-HA
PTD-HA
MSH5-HA
c
HEI10-Myc
HA-J3
HEI10-Myc
HA-J3G155R
PTD-Myc
HA-J3
PTD-Myc
HA-J3G155R
MSH5-Myc
HA-J3
MSH5-Myc
HA-J3G155R
IB: Myc
IB: HA
Ponceau
mRNA
d
Relative intensity
HA-J3
HA-J3G155R
HEI10-Myc
PTD-Myc
MSH5-Myc
e
Col
hcr3
j3-1
J3::J3G155R
IB: HEI10
IB: J3/J2
CB
f
Relative intensity
Col
hcr3
j3-1
J3::J3G155R

## INTERNATIONAL SEARCH REPORT

International application No.
**PCT/KR2024/012205**

**A. CLASSIFICATION OF SUBJECT MATTER**

**A01H 1/06**(2006.01)i; **C12N 15/82**(2006.01)i; **A01H 5/10**(2006.01)i

According to International Patent Classification (IPC) or to both national classification and IPC

**B. FIELDS SEARCHED**

Minimum documentation searched (classification system followed by classification symbols)

A01H 1/06(2006.01); A01H 5/00(2006.01); A01H 5/10(2006.01); A01H 6/82(2018.01); C12N 15/113(2010.01); C12Q 1/68(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 샤페론 단백질(chaperone protein), 저해제(inhibitor), 식물(plant), 감수분열(meiosis), 교차(crossover), 재조합(recombination)

**C. DOCUMENTS CONSIDERED TO BE RELEVANT**

| Category* | Citation of document, with indication, where appropriate, of the relevant passages | Relevant to claim No. |
|---|---|---|
| A | KR 10-2022-0022237 A (POSTECH RESEARCH AND BUSINESS DEVELOPMENT FOUNDATION) 25 February 2022 (2022-02-25)<br>See claims 1, 4 and 8. | 1-13 |
| A | KR 10-2022-0086041 A (SEOUL NATIONAL UNIVERSITY R&DB FOUNDATION) 23 June 2022 (2022-06-23)<br>See claims 1-3. | 1-13 |
| A | US 2009-0126039 A1 (SANZ MOLINERO, A. I.) 14 May 2009 (2009-05-14)<br>See claim 1; and SEQ ID NO: 3. | 1-13 |
| A | KR 10-2022-0086037 A (SEOUL NATIONAL UNIVERSITY R&DB FOUNDATION) 23 June 2022 (2022-06-23)<br>See abstract; and claim 1. | 1-13 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **27 November 2024** | **27 November 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT

International application No.
**PCT/KR2024/012205**

**C. DOCUMENTS CONSIDERED TO BE RELEVANT**

| Category* | Citation of document, with indication, where appropriate, of the relevant passages | Relevant to claim No. |
|---|---|---|
| A | KR 10-2022-0161666 A (INDUSTRY-ACADEMIC COOPERATION FOUNDATION GYEONGSANG NATIONAL UNIVERSITY) 07 December 2022 (2022-12-07) See claim 1. | 1-13 |

Form PCT/ISA/210 (second sheet) (July 2022)

**INTERNATIONAL SEARCH REPORT**

International application No.
**PCT/KR2024/012205**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/KR2024/012205**

| Patent document cited in search report | Publication date (day/month/year) | Patent family member(s) | Publication date (day/month/year) |
|---|---|---|---|
| KR 10-2022-0022237 A | 25 February 2022 | KR 10-2410996 B1 | 20 June 2022 |
| KR 10-2022-0086041 A | 23 June 2022 | KR 10-2528935 B1 | 03 May 2023 |
| US 2009-0126039 A1 | 14 May 2009 | AR 051866 A1 | 14 February 2007 |
| | | AT 528404 T | 15 October 2011 |
| | | AT E528404 T1 | 15 October 2011 |
| | | AU 2005-318112 A1 | 29 June 2006 |
| | | AU 2005-318112 B2 | 24 February 2011 |
| | | BR PI0519260 A2 | 06 January 2009 |
| | | CA 2594554 A1 | 29 June 2006 |
| | | CN 101128590 A | 20 February 2008 |
| | | CN 101128590 B | 02 January 2013 |
| | | EP 1833971 A1 | 19 September 2007 |
| | | EP 1833971 B1 | 12 October 2011 |
| | | ES 2375488 T3 | 01 March 2012 |
| | | MX 2007007389 A | 04 September 2007 |
| | | US 2011-0179527 A1 | 21 July 2011 |
| | | US 7872173 B2 | 18 January 2011 |
| | | WO 2006-067236 A1 | 29 June 2006 |
| | | ZA 200704606 B | 26 November 2008 |
| KR 10-2022-0086037 A | 23 June 2022 | KR 10-2598907 B1 | 03 November 2023 |
| KR 10-2022-0161666 A | 07 December 2022 | KR 10-2665616 B1 | 10 May 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 2410996
- EP 0116718 B1
- EP 0120516 B1
- US 4940838 A
- EP 0301316 A


### Non-patent literature cited in the description


- **KRENS, FA et al.** *Nature*, 1982, 72-74
- **NEGRUTIU I et al.** *Plant Mol Biol*, 1987, vol. 8, 363-373
- **SHILLITO RD et al.** *Bio/Technol*, 1985, vol. 3, 1099-1102
- **CROSSWAY A et al.** *Mol Gen Genet*, 1986, vol. 202, 179-185
- **KLEIN TM et al.** *Nature*, 1987, vol. 327, 70
- **WU, G. et al.** *Genetics*, 2015, vol. 200, 35-45
- **MELAMED-BESSUDO, C. et al.** *Plant J*, 2005, vol. 43, 458-66
- **YELINA, N. E. et al.** *Genes Dev.*, 2015, vol. 9, 2183-202
- **F. HARTUNG et al.** *Nucleic Acids Research*, 2006, vol. 34 (16), 4438-4448
- **BERCHOWITZ, L. E.** ; **COPENHAVER, G. P**. *Nat. Protoc.*, 2008, vol. 3, 41-50
- **LIM, E. C. et al.** *Plant J*, 2020, vol. 101, 473-483
- **CHELYSHEVA, L. et al.** *Cytogenet. Genome Res.*, 2010, vol. 29, 143-153
- **HWANG, I** ; **SHEEN, J**. *Nature*, 2001, vol. 413, 383-389